# EUROPÄISCHE PATENTSCHRIFT

(11) **EP 1 103 622 B1**
(45) Veröffentlichungstag und Bekanntmachung des Hinweises auf die Patenterteilung: **16.03.2005**
(21) Anmeldenummer: 00125818.5
(22) Anmeldetag: 24.11.2000
(51) Int. Cl.: C12Q 1/68

(54) **Spezies-spezifischer Nachweis von Nukleinsäuren mittels eines Analyseelements**
Species specific detection of nucleic acids using an analysing element
Détection d'acides nucléiques pour des espèces spécifiques utilisant un élément d'analyse

(30) Priorität: 25.11.1999 DE 19956820
(43) Veröffentlichungstag der Anmeldung: 30.05.2001
(73) Patentinhaber: Roche Diagnostics GmbH, 68305 Mannheim (DE)
(72) Erfinder: Keller, Volker, 54129 Bergisch Gladbach (DE); Rauscher, Andreas, 67071 Ludwigshafen (DE); Steinbiss, Joachim, 64653 Lorsch (DE); Schlipfenbacher, Reiner, 67098 Bad Dürkheim (DE); Klepp, Jürgen, 76131 Karlsruhe (DE)
(74) Vertreter: Weiss, Wolfgang, Dipl.-Chem. Dr.

(56) Entgegenhaltungen:
- EP-A- 0 926 498
- WO-A-95/27081
- US-A- 5 527 673
- US-A- 6 037 127
- DOERNER A ET AL: "Tissue-specific transcription pattern of the adenine nucleotide translocase isoforms in humans" FEBS LETTERS, ELSEVIER SCIENCE PUBLISHERS, AMSTERDAM, NL, Bd. 414, Nr. 2, 8. September 1997 (1997-09-08), Seiten 258-262, XP004366326 ISSN: 0014-5793
- CERDA S R ET AL: "Altered expression of the DNA repair protein, N-methylpurine-DNA glycosylase (MPG), in breast cancer" FEBS LETTERS, ELSEVIER SCIENCE PUBLISHERS, AMSTERDAM, NL, Bd. 431, Nr. 1, 10. Juli 1998 (1998-07-10), Seiten 12-18, XP004258932 ISSN: 0014-5793

## Beschreibung

Die vorliegende Erfindung betrifft ein Verfahren zum Nachweis von Nukleinsäuren auf einem Analyseelement, das eine Probenauftragzone und eine Nachweiszone enthält, wobei das Analyseelement einen Flüssigkeitstransport von der Probenauftragzone zur Nachweiszone ermöglicht, wobei eine Probe auf die Probenauftragzone des Analysenelements aufgegeben und die in der Probe enthaltenen Nukleinsäuren in der Nachweiszone durch Hybridisierung mit einer Nachweissonde qualitativ, aber auch quantitativ vorzugsweise über Markierungsgruppen nachgewiesen werden können. Weiterhin werden neue Analyseelemente und Reagenzienkits zum Nachweis von Nukleinsäuren bereitgestellt.

Der gängige Nachweis von Nukleinsäuren beispielsweise Amplifikationsprodukten aus einer PCR, im Rahmen diagnostischer Anwendungen erfolgt über die spezifische Hybridisierung der nachzuweisenden Nukleinsäure mit einer Nachweissonde, üblicherweise einem Oligonukleotid und einer nicht radioaktiven Nachweisreaktion über Markierungsgruppen, die in den nachzuweisenden Nukleinsäuren oder/und der Nachweissonde vorliegen können. Für diese Nachweisreaktion gibt es verschiedene Verfahrensvarianten. So kann eine direkte Hybridisierung der nachzuweisenden Nukleinsäuren mit einer an einer Festphase, beispielsweise einer Mikrotiterplatte, immobilisierten Nachweissonde erfolgen (Amplicor Detection Kit, Roche Diagnostics, EP-A-0 420 260). Weiterhin kann die nachzuweisende Nukleinsäure mit einer markierten Nachweissonde vorhybridisiert und der dabei entstehende Komplex an einer Festphase, beispielsweise einer Mikrotiterplatte, abgefangen werden (PCR-Detection Kit, Roche Diagnostics).

Bei herkömmlichen Southern-Blot- bzw. Northern-Blot-Techniken (siehe z.B. Cerda et al., FEBS Letters 431 (1998), 12-18; Doerner et al., FEBS Letters 414 (1997), 258-262) erfolgt zunächst eine Auftrennung von Nukleinsäuren mittels Gelelektrophorese. Nach Denaturierung werden die Nukleinsäuren auf eine Nylonmembran übertragen, dort fixiert und durch Hybridisierung mit markierten Hybridisierungssonden, insbesondere mit radioaktiv markierten Hybridisierungssonden nachgewiesen.

Die Verwendung von Teststreifen in diagnostischen Verfahren ist seit langem bekannt. So ist der Nachweis von Analyten mit Teststreifen in EP-B-0 186 799, EP-B-0 262 328 und EP-A-0 926 498 beschrieben, die vornehmlich immunologisch ausgerichtete Testformate betreffen.

Jou et al. (Human Mutation 5 (1995), 86-93) beschreiben ein Verfahren zum Nachweis von Nukleinsäuren auf chromatographischen Teststreifen, wobei in die nachzuweisenden Nukleinsäure Hapten-Gruppen eingeführt werden, so daß eine immunologische Immobilisierung der nachzuweisenden Nukleinsäuren auf dem Teststreifen über Anti-Hapten-Antikörper möglich wird. Ein Abfangen nachzuweisender Nukleinsäuren über spezifische Hybridisierung an immobilisierte Nachweissonden auf Teststreifen wird beispielsweise in US 5,310,650, EP-B-0 612 354 und Reinhartz et al. (Gene 136 (1993), 221-226) und Rule et al. (Clin. Chem. 42 (1996), 1206-1209) beschrieben.

Die zuvor genannten Verfahren, in denen der Nachweis von Nukleinsäuren auf Teststreifen beschrieben ist, weisen jedoch eine Reihe Nachteile auf. So verringert sich bei einer Immobilisierung der Nukleinsäure über Sandwich-Komplexe die Sensitivität. Einer Sensitivitätssteigerung beispielsweise über Erhöhung des Nachweisvolumens ist aufgrund des begrenzten Einbaus von Markierungsgruppen in die nachzuweisenden Nukleinsäuren Grenzen gesetzt.

Mit den genannten Verfahren können die Nukleinsäuren jedoch nicht unmittelbar nachgewiesen werden. In einem vorgeschalteten Schritt wird zuerst der Komplex zwischen Zielnukleinsäure und Hybridisierungssonde gebildet. Hierfür wird die Zielnukleinsäure denaturiert (thermisch oder durch Zugabe einer alkalischen Lösung), falls nötig neutralisiert, die Sonde zugegeben und Hybridisierungsbedingungen eingestellt. Erst im Anschluß an diese Vorprozessierung kann der eigentliche Nachweis auf dem Testträger erfolgen. Da diese Vorprozessierung mit dosiertenReagenzzugaben und der Benutzung von Geräten, z.B. Temperiereinrichtungen, verbunden ist, können die wesentlichen Vorteile von Teststreifen-basierten Verfahren, wie geringer Gerätebedarf, einfache Handhabung, wenige Arbeitsschritte und der Durchführbarkeit des Verfahrens von ungeschulten Personen nicht genutzt werden. Weiterhin ergeben sich durch diese zusätzlichen Schritte Kontaminationsrisiken, die beim Nachweis amplifizierter Nukleinsäuren zu falsch-positiven Ergebnissen führen können.

Auch bei Verfahren, die eine direkte Hybridisierung der nachzweisenden Nukleinsäure an die Hybridisierungssonde auf dem Analyseelement erlauben, sind im Stand der Technik keine sensitiven Nachweisverfahren bekannt, die auf eine Vorprozessierung der nachzuweisenden Nukleinsäure verzichten, so daß auch bei diesem Verfahren die Nukleinsäure nicht direkt, bzw. nicht direkt nach einem Amplifikationsprozess nachgewiesen werden kann. Bei diesen Verfahren wird die, im Regelfall doppelsträngig vorliegende, Zielnukleinsäure für die Hybridisierung in eine einzelsträngige Konformation überführt. Hierfür sind mehrere Verfahren beschrieben. So kann nach allgemein bekannten Hybridisierungsverfahren die Nukleinsäure vor Aufgabe auf das Analyseelement vollständig denaturiert werden, z.B. thermisch oder durch Zugabe alkalischer Reagenzien. Weiterhin sind auch enzymatische Verfahren bekannt, die einen gezielten Verdau eines Stranges der Zielnukleinsäure ermöglichen (Rule et al. Clinical Chemistry 42, S. 1206-1209 (1996)).

Auf eine Vorprozessierung kann nur dann verzichtet werden, wenn die Zielnukleinsäuren bereits vollständig einzelsträngig vorliegen. So ergeben sich jedoch üblicherweise bereits beim Nachweis von ribosomalen Zielnukleinsäuren Schwierigkeiten, weil diese einzelsträngigen Nukleinsäuren in ihrer nativen Sekundärstruktur in goßen Bereichen doppelsträngig vorliegen (beispielsweise rRNA). Eine theoretische Alternative sind Amplifikationsverfahren, die eine gezielte Synthese nur eines Nukleinsäurestranges erlauben. Ein Beispiel hierfür ist die asymmetrische PCR. Da dieses Verfahren aufgrund der linearen Amplifikation jedoch deutlich ineffizienter ist als konventionelle PCR-Verfahren, ist die Sensitivität deutlich eingeschränkt und beim Nachweis von in nur geringer Konzentration vorliegenden Analyten unzureichend. Somit zeigen auch im Stand der Technik bekannte Verfahren, die eine direkte Hybridisierung der nachzuweisenden Nukleinsäure auf einem Analyseelement erlauben, die gleichen Nachteile, wie sie bei den indirekten Nachweisverfahren festzustellen sind.

Ein wesentlicher Vorteil der Erfindung liegt darin, daß die zuvor beschriebene Vorprozessierung der nachzuweisenden Nukleinsäure entfällt und die Nukleinsäuren direkt, bzw. im Anschluß an einen Amplifikationsprozess auf ein Analyseelement aufgegeben werden können. Die auf dem Analyseelement bevorzugt imprägniert vorliegenden Reagenzien ermöglichen eine vollständige, bzw. partielle Denaturierung der Zielnukleinsäure auf dem Analyseelement, so daß auch eine Hybridisierung von in doppelsträngiger Konformation aufgebrachten Nukleinsäuren ermöglicht wird. Hierfür bestehen erfindungsgemäß mehrere Möglichkeiten. So können auf dem Testträger denaturierende Reagenzien in einer geeigneten Konzentration und bevorzugt in getrockneter Form vorgelegt werden, um eine vollständige Denaturierung der Nukleinsäuren zu bewirken. Die Zielnukleinsäure kann in flüssiger Form, beispielweise in der PCR-Reaktionslösung vorliegend, auf das Analyseelement aufgetragen werden. Nach Anlösen der Reagenzien wird die Zielnukleinsäure vollständig denaturiert. Da unter diesen Bedingungen eine Hybridisierung an das Sondenoligonukleotid nicht erfolgen kann, ist während des bevorzugt chromatographischen Transports des Analyten über den Testräger das chemische Milieu entsprechend zu ändern. Dies kann einerseits durch entsprechende Chromatographiepuffer geschehen oder durch stromabwärts vorgelegte Reagenzien. Bevorzugt hierfür ist eine Denaturierung mittels einer Base, insbesondere NaOH und die Neutralisierung mittels einer Säure. Alternativ kann das chemische Milieu so eingestellt werden, daß die doppelsträngige Konformation der Zielnukleinsäure destabilisiert (partiell denaturiert) wird, und die Hybridisierung an das Sondenoligonukleotid ermöglicht, bzw. unterstützt wird. Eine Veränderung des chemischen Milieus während der Chromatographie ist bei dieser Verfahrensvariante nicht nötig. Bevorzugte Reagenzien für diese Ausführungsform sind chaotrope Salze, besonders bevorzugt Guanidiniumthiocyanat (GuSCN).

Um die, an das Sondenoligonukleotid hybridisierten Zielnukleinsäuren nachweisen zu können, ist weiterhin die Markierung des Komplexes nötig. Die Markierungsgruppen können sowohl über einen enzymatischen Einbau während eines optional vorgschalteten Amplifikationsverfahrens in die amplifizierte Zielnukleinsäure eingebaut werden oder mittels Hybridisierung eines weiteren entsprechend markierten Sondenoligonukleotids an die Zielnukleinsäure binden.

Überraschenderweise wurde festgestellt, daß bei Verwendung des oben beschriebenen Verfahrens Zielnukleinsäuren, welche in doppelsträngiger Form auf das Analyseelement aufgetragen werden, trotz der nur sehr kurzen, während des chromatographischen Transports der Zielnukleinsäure über das Analyseelement zur Verfügung stehenden Zeit sehr effektiv an das Sondenoligonukleotid hybridisieren und mithoher Sensitivität nachgewiesen werden können.

Ein Gegenstand der Erfindung ist somit ein Verfahren zum Nachweis von Nukleinsäuren auf einem Analyseelement, das eine Probenauftragzone und eine Nachweiszone enthält, wobei das Analyseelement einen Flüssigkeitstransport von der Probenauftragzone zur Nachweiszone ermöglicht, umfassend die Schritte:
- Aufgeben einer die nachzuweisenden Nukleinsäuren enthaltenden Probe auf die Probenauftragzone,
- Nachweisen der Nukleinsäure in der Nachweiszone durch Hybridisierung mit einer Nachweissonde,
dadurch gekennzeichnet, daß die nachzuweisenden Nukleinsäuren auf dem Analyseelement denaturiert werden.

Unter "Denaturierung" soll hier nicht nur eine vollständige Denaturierung im klassischen Sinn verstanden werden, sondern es soll auch eine partielle Denaturierung mit umfaßt sein, bei der ein Nukleinsäuredoppelstrang soweit destabilisiert wird, daß eine Hybridisierung mit einer Sonde stattfinden kann. Es ist also keine vollständige Auflösung des Doppelstrangs notwendig, obwohl natürlich auch eine solche Ausführungsform erfindungsgemäß mit umfaßt ist.

Erfindungsgemäß erfolgt eine Denaturierung der nachzuweisenden Nukleinsäuren auf dem Analyseelement, d.h. die Nukleinsäuren werden in nichtdenaturierter, vorzugsweise doppelsträngiger Form auf das Analyseelement aufgebracht und kommen mit dem Denaturierungsreagenz erst nach Auftrag auf das Analyseelement in Kontakt. Das Denaturierungsreagenz kann eine Base oder/und eine chaotrope Substanz in einer Menge, die zur Denaturierung von in der Probe vorhandenen Nukleinsäuren ausreicht, enthalten. Bevorzugte Beispiele für Basen sind Alkalihydroxide, insbesondere NaOH. Bevorzugte Beispiele für chaotrope Substanzen sind lodide, Acetate, Perchlorate, Thiocyanate, Trifluoracetate, Trichloracetate oder/und Guanidiniumverbindungen. Besonders bevorzugtistGuanidiniumthiocyanat. Durch Verwendung chaotroper Substanzen wird einerseits eine Doppelstrang-destabilisierende Wirkung erzielt und eine Beschleunigung der Hybridisierung zwischen nachzuweisenden Nukleinsäuren und Nachweissonde errreicht. Eine weitere wichtige Funktion liegt in der Absenkung des spezifischen Schmelzpunktes der Nukleinsäure, die eine spezifische Hybridisierung bei Raumtemperatur erlaubt.

Diese Wirkung chaotroper Substanzen ist für Hybridisierungen in Lösungen bereits bekannt (WO-A-87 06621, Thompson und Gillespie, Anal. Biochem. 163 (1987), 281-291; Van Ness und Chen, Nucleic Acid Res. 19 (1991), 5143-5151), wird im Rahmen der vorliegenden Erfindung jedoch erstmals für den Nachweis von Nukleinsäuren durch Hybridisierung auf Analyseelementen genutzt. Eine Übertragung dieser Technik auf ein Analysenelement hat sich für den Fachmann nicht in naheliegender Weise aus dem Stand der Technik ergeben, weil dort Hybridisierungszeiten in der Größenordnung von etwa einer Stunde als erforderlich empfohlen werden. Bei erfindungsgemäßen Analyseelementen ist die Untersuchung von der Probenaufgabe bis zur Detektion jedoch bereits nach weniger als 10 min abgeschlossen. Die Hybridisierung mit der Nachweissonde erfolgt während des chromatographischen Transports der Probe über die Nachweiszone, so daß der Kontakt zwischen Sonde und Probe nur wenige Sekunden, auf jeden Fall aber weniger als 1 min, beträgt.

Wesentlich für das erfindungsgemäße Analysenelement ist, daß sich Flüssigkeit innerhalb des Analyseelements von der Probenauftragzone in Richtung auf die Nachweiszone bewegen kann. Ein solcher Flüssigkeitsstrom ist beispielsweise in einem entsprechend hergerichteten Hohlkörper durch Schwerkraft möglich. Vorrichtungen, die einen Flüssigkeitstransport durch Zentrifugalkraft als einer Form der Schwerkraft ermöglichen, sind beispielsweise aus EP-B-0 052 769 bekannt. Bevorzugt enthalten erfindungsgemäße Analyseelemente jedoch saugfähige Materialien, die Flüssigkeit durch Kapillarkraft zu bewegen vermögen. Die Materialien der einzelnen Zonen des erfindungsgemäßen Analyseelements können hierbei gleich oder auch verschieden sein. Häufig wird es so sein, daß unterschiedliche Zonen aus unterschiedlichen Materialien bestehen, wenn diese optimal ihre Aufgabe erfüllen sollen.

Als mögliche saugfähige, kapillaraktive Materialien kommen grundsätzlich alle solche in Frage, die generell in sogenannten "Trockentesten", wie sie beispielsweise in US-A-4,861,711, US-A-5,591,645 oder EP-A-0 291 194 beschrieben sind, zur Flüssigkeitsaufnahme eingesetzt werden können. Als vorteilhaft haben sich hierfür beispielweise poröse Materialien, wie Membranen, beispielweise Nitrocellulosemembranen erwiesen. Es können jedoch auch faserige, saugfähige Matrixmaterialien wie Vliese, Gewebe oder Gewirke eingesetzt werden. Vliese sind besonders bevorzugt. Faserige Matrixmaterialien können Glas, Cellulose, Cellulosederivate, Polyester Polyamid, aber auch Viskose, Zellwolle oder/und Polyvinylalkohol enthalten. Vliese aus Fasern auf der Basis von Cellulose, Polymerisatfasern auf Basis von Polyester und/oder Polyamid und einem organischen Bindemittel, das OH- und/oder Estergruppen aufweist, wie sie aus EP-B-0 326 135 bekannt sind, sind beispielsweise erfindungsgemäß einsetzbar. Vliesmaterialien, enthaltend schmelzbare Copolyesterfasern, Cellulosefasern oder Cellulosederivatefasern, wie sie in der europäischen Patentanmeldung 0 571 941 beschrieben sind, können auch in dem erfindungsgemäßen Analyselement eingesetzt werden. Papiere, wie beispielsweise Teebeutelpapier, sind ebenfalls gut einsetzbar.

Zur Verbesserung der Handhabung des erfindungsgemäßen Analyseelements kann sich das saugfähige kapillaraktive Material oder können sich unterschiedliche saugfähige, kapillaraktive Materialien auf einem steifen Trägermaterial angeordnet befinden, welches seinerseits für Flüssigkeit nicht durchlässig ist, den Flüssigkeitsfluß im Matrixmaterial aber nicht negativ beeinflußt und sich in Bezug auf die im Analyseelement ablaufenden Reaktionen inert verhält. Bevorzugtes Trägermaterial kann beispielsweise Polyester-Folie sein, auf der das den Flüssigkeitstransport ermöglichende Matrixmaterial befestigt ist.

In dem erfindungsgemäßen Analyseelement können die einzelnen Zonen übereinander, nebeneinander oder teils übereinander und teils nebeneinander auf dem Trägermaterial angeordnet sein. Besonders bevorzugt ist ein erfindungsgemäßes Analyseelement, bei dem sich Probenauftrag- und Nachweiszone nebeneinander auf dem Trägermaterial angeordnet befinden. Nebeneinander bedeutet in diesem Zusammenhang, daß sich diese Zonen in direktem Kontakt miteinander befinden oder aber durch andere Zonen getrennt im wesentlichen in einer Ebene angeordnet sind.

Die Probenauftragzone ist der Bereich des erfindungsgemäßen Analyselementes, auf den die Probe aufgegeben wird. Die Nachweiszone ist der Bereich des erfindungsgemäßen Analyseelements, in dem die Bestimmung erfolgt, ob der untersuchte Analyt bzw. die vom Analyt abgeleitete und diesen repräsentierende Substanz in der auf das Analyseelement gegebenen Probe vorhanden war. Diese Bestimmung kann qualitativ, halb quantitativ oder quantitativ sein. Halb quantitativ bedeutet hierbei, daß für den Analyten bzw. die vom Analyten abgeleitete und diesen repräsentierende Substanz kein konkreter Konzentrationswert, sondern ein Konzentrationsbereich ermittelt wird, in dem sich die Analytkonzentration befindet.

In einer ersten Ausführungsform der Erfindung wird das Analyseelement vor Aufgeben der Probe mit dem Denaturierungsreagenz getränkt. Dabei kann das Denaturierungsreagenz auf dem Analyseelement, vorzugsweise auf dessen Probenauftragzone, in flüssiger oder trockener Form vorliegen. Besonders bevorzugt liegt das Denaturierungsreagenz in trockener Form vor. In einer weiteren Ausführungsform kann das Analyseelement jedoch auch erst nach dem Aufgeben der Probe mit dem Denaturierungsreagenz getränkt werden. Aufgrund des Kontakts der in der nachzuweisenden Probe vorhandenen Nukleinsäuren mit dem Denaturierungsreagenz wird eine Denaturierung von Nukleinsäuredoppelsträngen bewirkt, wodurch eine Hybridisierung der resultierenden Nukleinsäureeinzelstränge mit der Nachweissonde ermöglicht wird. Sofern das Denaturierungsreagenz eine Base enthält, kann es unter Umständen zweckmäßig sein, daß das Analyseleement zwischen Probenauftragszone und Nachweiszone ein Neutralisierungsreagenz, z.B. eine bei Kontakt mit Flüssigkeiten sauer reagierendes Salz wie ein Hydrogenphosphat oder ein Hydrogensulfat, vorzugsweise in trockener Form, enthält.

Die zur Bindung von in der Probe vorhandenen nachzuweisenden Nukleinsäuren verwendete Nachweissonde ist vorzugsweise ein Oligonukleotid, welches eine für die nachzuweisenden Nukleinsäuren ausreichend komplementäre Sequenz enthält, um eine Hybridisierung unter den Testbedingungen zu ermöglichen. Vorzugsweise ist die Nachweissonde ein Oligodesoxyribonukleotid, es können jedoch auch Nukleinsäureanaloga mit modifizierten Nukleotidbausteinen oder peptidische Nukleinsäuren als Nachweissonden verwendet werden. Die Nachweissonde liegt vorzugsweise in der Nachweiszone in immobilisierter Form vor, d.h. sie wird unter Testbedingungen im wesentlichen nicht aus der Nachweiszone eluiert. Die Immobilisierung kann durch Auftrag der Nachweissonde und anschließende Fixierung mittels geeigneter Methoden, z.B. durch Temperaturerhöhung, Bestrahlung etc. erfolgen. Alternativ kann die Sonde auch mittels hochaffiner Wechselwirkungen, z.B. Biotin/Streptavidin bzw. Avidin oder Hapten/Anti-Hapten-Antikörper, Zucker/Lectin in der Nachweiszone immobilisiert werden. Die Herstellung entsprechend modifizierter, z.B. biotinylierter Nachweissonden ist dem Fachmann geläufig und muß hier nicht näher ausgeführt werden. Alternativ kann die Hybridisierung zwischen nachzuweisenden Nukleinsäuren und Nachweissonde auch stromaufwärts der Nachweiszone erfolgen und der Hybridisierungskomplex selektiv, z.B. mittels hochaffiner Wechselwirkungen wie zuvor beschrieben, beim Transport durch das Analyseelement in der Nachweiszone fixiert werden.

Der Nachweis der Nukleinsäuren erfolgt vorzugsweise über Markierungsgruppen, wobei grundsätzlich alle aus dem Stand der Technik bekannten Markierungsgruppen geeignet sind. Bevorzugt sind visuell erkennbare Markierungsgruppen wie Enzym-Markierungen, insbesondere jedoch partikuläre Markierungen wie Gold- oder Latexpartikel. Die Markierung kann dabei unmittelbar auf den nachzuweisenden Nukleinsäuren lokalisiert sein (direkte Markierung), sie kann jedoch auch mittelbar, z.B. über hochaffine Wechselwirkungen wie zuvor beschrieben, beispielsweise über Hapten/Anti-Hapten-Antikörper an die Nukleinsäuren gebunden werden (indirekte Markierung). Die Bindung indirekter Markierungsgruppen an die nachzuweisenden Nukleinsäuren kann während der Hybridisierung oder erst nach einem Waschschritt erfolgen.

Durch die vorliegende Erfindung wird ein einfaches Verfahren zum Nachweis von Nukleinsäuren auf Teststreifen bereitgestellt. Bei den nachzuweisenden Nukleinsäuren handelt es sich vorzugsweise um Amplifkationsprodukte, z.B. Amplifikationsprodukte, die durch eine PCR erzeugt wurden. Dabei können die Amplifikationsprodukte unmittelbar unter Verwendung der Pufferlösung, in der die Amplifikationsreaktion durchgeführt worden war, als chromatographisches Laufmittel auf dem Teststreifen aufgetragen werden.

Ein weiterer Gegenstand der Erfindung ist ein Analyseelement zum Nachweis von Nukleinsäuren, das eine Probenauftragzone und eine Nachweiszone enthält, wobei das Analyseelement einen Flüssigkeitstransport von der Probenauftragzone zur Nachweiszone ermöglicht, das mit einem Denaturierungsreagenz für Nukleinsäuren getränkt ist, wobei das Denaturierungsreagenz vorzugsweise in trockener Form vorliegt. Das Analyseelement kann ein saugfähiges Material enthalten, welches einen chromatographischen Transport ermöglicht, und ist vorzugsweise als Teststreifen ausgebildet. In der Nachweiszone des Analyselements ist vorzugsweise eine zu den nachzuweisenden Nukleinsäuren komplementäre Nachweissonde in immobilisierter Form vorhanden.

Schließlich betrifft die Erfindung einen Kit zum Nachweis von Nukleinsäuren umfassend ein erfindungsgemäßen Analyseelement und weitere zum Nachweis von Nukleinsäuren erforderliche Reagenzien, z.B. Markierungsreagenzien, Puffer etc.

Die durch die vorliegende Erfindung bereitgestellten Verfahren, Analysenelemente und Reagenzienkits eignen sich besonders zum Nachweis von Nukleinsäure-Amplifikationsprodukten, z.B. zum Nachweis des Vorhandenseins oder der Menge von ausgewälten Organismen oder Teilen von Organismen, z.B. Zellen in einer Probe. Hierzu kann beispielsweise ein Gattungs- und Spezies-spezifischer Nachweis von Nukleinsäuren in einer Probe, z.B. durch Amplifkation von ribosomaler RNA oder einer dafür kodierenden DNA-Sequenz von in einer Probe vorkommenden Organismen oder Zellen erfolgen. Das Verfahren kann zum Nachweis von Mikroorganismen, z.B. von pathogenen Mikroorganismen oder Viren in klinischen Proben, Lebensmitteln, Gewässerproben etc. eingesetzt werden. Weiterhin kann das Verfahren auch forensische Analyse zum Nachweis von spezifischen Individuen eingesetzt werden. In den nachfolgenden Beispielen ist der Nachweis von Chlamydia trachomatis stammenden Nukleinsäuren durch das erfindungsgemäße Verfahren gezeigt.

### Figurenbeschreibung:

- Figur 1: zeigt den schematischen Aufbau eines erfindungsgemäßen Teststreifens mit einer Probenauftragzone (1), die als Saugvlies ausgebildet ist, einer Flüssigkeitstransportzone (2) mit einer darin befindlichen Nachweiszone (4) und einer Saugzone (3), die ebenfalls als Saugvlies ausgebildet ist.
- . Figur 2: zeigt einen Vergleich zwischen der Sensitivität des erfindungsgemäßen Verfahren (B) und dem in EP-A-0 926 498 beschriebenen Verfahren (A).
- Figur 3: zeigt die Sensitivität eines Analyseelementes in Abhängigkeit von der Guanidiniumthiocyanat (GuSCN)-Konzentration.

### Beispiel 1 Konzeption

### 1. Vorbereitung des Analyseelements

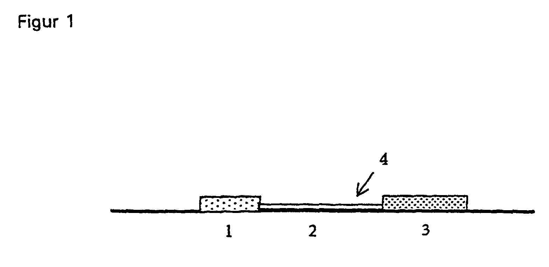

Das Saugvlies (1) des in Figur 1 gezeigten Analyseelements wurde mit Tränkpuffer (10 mM KPO₄ pH 7,5, 100 mM NaCI, 2 oder 2,5 M Guanidiniumthiocyanat (GuSCN), 0,5% Triton-X100, 25 mM NaOH, 2% RPLA Typ 4 (Roche Diagnostics, Kat.-Nr. 1726544-106) und 3 mg/ml Heringssperma-DNA) getränkt. Hierzu wurde das Saugvlies vollständig in einer Petrischale mit Tränkpuffer benetzt und 3 h bei 45°C unter Umluft getrocknet.

Als Flüssigkeittransportzone (2) wurde eine 15 mm Nitrozellulosemembran verwendet. Zur Herstellung der Nachweiszone (4) wurden Nachweisoligonukleotidsonden (40 pmol pro Teststreifen) auf die Membran aufgetragen. Dabei wurde eine Lösung der Oligonukleotide ( 200 µM) in Form eines Nachweisstrichs über einen automatischen Dispensor (Hamilton Microlab N) aufgebracht und die Oligonukleotide auf der Membran bei 80°C für 30 min fixiert. Alternativ wurden Biotin-markierte Oligonukleotide auf eine mit Polystreptavidin vorbeschichtete Nachweiszone (4) aufgebracht. Hierzu wurde eine Oligonukleotidlösung (8 pmol/Testreifen) auf die Nitrozellulosemembran aufgegeben und mit 10 µl Standardchromatographiepuffer über die Polystreptavidin-vorbeschichtete Nachweiszone chromatographiert.

Als Nachweissonde wurde ein Oligonukleotid mit der Sequenz 5'-GTC TCT CAT CGA GAC AAA GTG-3' aus dem Chlamydia trachomatis Plasmid pCTT1 (C. trachomatis Basen 1-7496) entsprechend Position 354-374 von pCTT1 verwendet (Sriprakash und Macavoy, Plasmid 18 (1987), 205-214).

### 2. PCR-Protokoll

Als Primer für die Amplifikation wurden die Oligonukleotide CP24 5'-GGGATTCCTGTAACAACAAGTCAGG-3' (Position 195-219 von pCTT1) und CP27: 5'-CCTCTTCCCCAGAACAATAAGAACAC-3' (Position 401-376 von pCTT1) gegebenenfalls im 5'-biotinylierter oder 5'-digoxigenylierter Form verwendet.

Das Reaktionsvolumen war 100 *µ*l (4 mM MgCl₂, jeweils 0,1 mM dNTP, jeweils 300 nM Primer CP24 und CP27, 2,5 U Taq-Polymerase, 2 U UNG (Uracil-DNA-Glycosylase) und Matrize in PCR-Puffer (Roche Diagnostics Katalog-Nr. 1600753).

Die Reaktionsführung war wie folgt:
- 10 min 37°C, 5 min bei 95°C, 1 min bei 60°C
- 34 Zyklen mit jeweils 30 s bei 95°C und 60 s bei 60°C
- 10 min bei 72°C
- Halten bei 50°C

Die PCR-Reaktion wurde mit unterschiedlichen Mengen an Matrize (0 bis 100 000 Plasmidkopien pro Ansatz) durchgeführt.

### 3. Nachweis

50 µl Amplifikat (Beispiel 2) wurden auf das Saugvlies des Teststreifens aufgegeben und für 4 min chromatographiert. Anschließend wurden 30 *µ*l Standardchromatographiepuffer (0,15 M NaCI, 9,6 mM KH₂PO₄, 40,5 mM K₂HPO₄, 0,25% Tween 20, 2% RPLA Typ 4, 0,09% Natriumazid) aufgegeben und 4 min chromatographiert. Anschließend wurden 20 *µ*l Anti-Digoxigenin-Antikörper-Gold-Konjugatlösung (2,1 pmol) aufgegeben und für 4 min chromatographiert. Anschließend wurden 2 mal jeweils 50 *µ*l Standardchromatographiepuffer aufgegeben und 4 min chromatographiert.

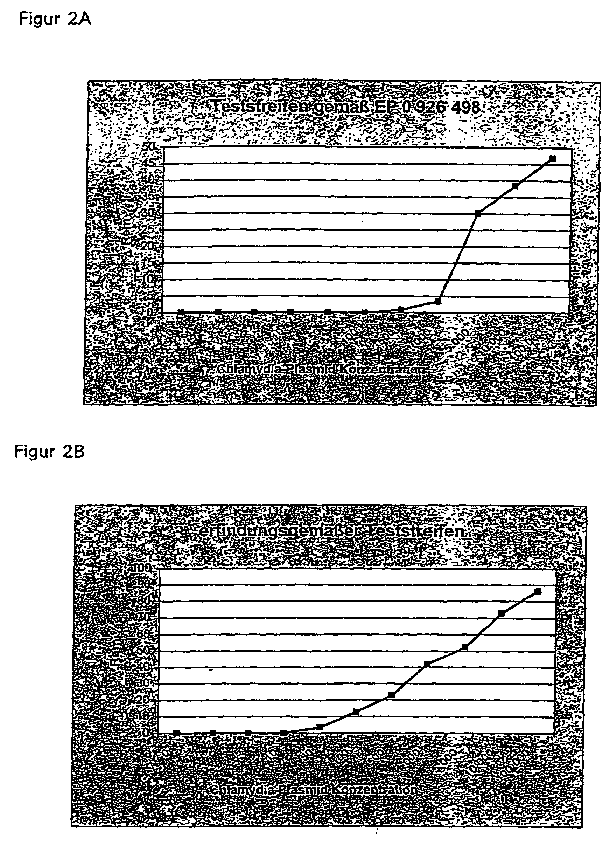

Die Ergebnisse dieses Versuchs sind in Figur 2 dargestellt. Figur 2A zeigt das Ergebnis mit einem Teststreifen gemäß EP-A-0 926 489, bei dem der Nachweis der Nukleinsäure durch immunologische Methoden mittels eines Sandwich-Formats erfolgt. Figur 2B zeigt das Ergebnis eines erfindungsgemäßen Nachweisverfahrens mit einem Teststreifen gemäß Beispiel 1. Während bei dem Teststreifen gemäß Figur 2A eine geringe Sensitivität (Nachweis erst ab > 1000 Kopien Plasmid pro Ansatz) gefunden wird, ist beim erfindungsgemäßen Verfahren ein Nachweis bereits bei einer erheblich geringeren Plasmidkopienzahl (50) möglich. Im allgemeinen ist ein positiver Nachweis bei Δ Rem-% ≥ 3 gegeben. Neben höherer Sensitivität zeigt das erfindungsgemäße Verfahren auch einen wesentlich größeren dynamischen Bereich, d.h. eine deutliche Signaländerung über einen großen Konzentrationsbereich der nachzuweisenden Nukleinsäure.

### Beispiel 2

Entsprechend Beispiel 1.1, mit der Ausnahme, daß im Tränkpuffer des Saugvlieses kein NaOH enthalten war, wurden Analyseelemente gemäß Fig. 1 hergestellt. Es wurden Guanidiniumkonzentrationen von 0,5 M, 1,5 M und 2,5 M (nahe der Sättigungsgrenze) getestet.

Auf die Analyseelemente wurde jeweils 50 µl PCR-Amplifikat (100 Plasmide pro Ansatz) aufgegeben, das entsprechend Beispiel 1.2 hergestellt worden war. Die Detektion erfolgte entsprechend Beispiel 1.3.

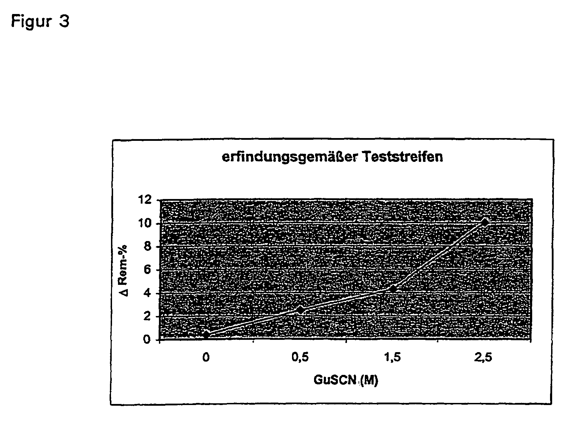

Die Ergebnisse sind in Fig. 3 dargestellt. Es ist hieraus ersichtlich,.daß der Zusatz von Guanidiniumthiocyanat die Nachweissensitivität signifikant verbessert.

## Patentansprüche

1. Verfahren zum Nachweis von Nukleinsäuren auf einem Analyseelement, das eine Probenauftragzone und eine Nachweiszone enthält, wobei das Analyselement einen Flüssigkeitstransport von der Probenauftragzone zur Nachweiszone ermöglicht, umfassend die Schritte:
- Aufgeben einer die nachzuweisenden Nukleinsäuren enthaltenden Probe auf die Probenauftragzone,
- Nachweisen der Nukleinsäuren in der Nachweiszone durch Hybridisierung mit einer Nachweissonde,
**dadurch gekennzeichnet,**
**daß** die nachzuweisenden Nukleinsäure erst nach dem Aufgeben auf das Analyseelement denaturiert werden.

2. Verfahren nach Anspruch 1,
**dadurch gekennzeichnet,**
**daß** die Nukleinsäuren durch Kontakt mit einem Denaturierungsreagenz enthaltend eine Base oder/und eine chaotrope Substanz denaturiert werden.

3. Verfahren nach Anspruch 2,
**dadurch gekennzeichnet,**
**daß** die Base aus Alkalihydroxiden, insbesondere NaOH, ausgewählt wird.

4. Verfahren nach Anspruch 2,
**dadurch gekennzeichnet,**
**daß** die chaotrope Substanz aus lodiden, Acetaten, Perchloraten, Thiocyanaten, Trifluoracetaten, Trichloracetaten oder/und Guanidiniumverbindungen ausgewählt wird

5. Verfahren nach Anspruch 4,
**dadurch gekennzeichnet,**
**daß** die chaotrope Substanz Guanidiniumthiocyanat ist.

6. Verfahren nach einem der vorhergehenden Ansprüche,
**dadurch gekennzeichnet,**
**daß** das Analyseelement ein saugfähiges Material enthält, das einen chromatographischen Transport ermöglicht.

7. Verfahren nach einem der Ansprüche 1 bis 6,
**dadurch gekennzeichnet,**
**daß** das Analyseelement vor dem Auftragen der Probe mit dem Denaturierungsreagenz getränkt wird.

8. Verfahren nach Anspruch 7,
**dadurch gekennzeichnet,**
**daß** das Denaturierungsreagenz auf dem Analyseelement in trockener Form vorliegt.

9. Verfahren nach Anspruch 7 oder 8,
**dadurch gekennzeichnet,**
**daß** die Probenauftragzone mit dem Denaturierungsreagenz vorgetränkt wird.

10. Verfahren nach einem der Ansprüche 1 bis 6,
**dadurch gekennzeichnet,**
**daß** das Analyseelement nach dem Aufgeben der Probe mit dem Denaturierungsreagenz getränkt wird.

11. Verfahren nach einem der vorhergehenden Ansprüche,
**dadurch gekennzeichnet,**
**daß** das Analyseelement zwischen Probenauftragzone und Nachweiszone ein Neutralisierungsreagenz enthält.

12. Verfahren nach einem der vorhergehenden Ansprüche,
**dadurch gekennzeichnet,**
**daß** die nachzuweisenden Nukleinsäuren Amplifikationsprodukte sind.

13. Verfahren nach einem der vorhergehenden Ansprüche,
**dadurch gekennzeichnet,**
**daß** die Nachweissonde in der Nachweiszone in immobilisierter Form vorliegt.

14. Verfahren nach einem der vorhergehenden Ansprüche,
**dadurch gekennzeichnet,**
**daß** der Nachweis der Nukleinsäuren über Markierungsgruppen erfolgt.

15. Verfahren nach Anspruch 14,
**dadurch gekennzeichnet,**
**daß** man indirekte Markierungsgruppen verwendet.

16. Analyseelement zum Nachweis von Nukleinsäuren, das eine Probenauftragszone und eine Nachweiszone enthält, wobei das Analyseelement einen Flüssigkeitstransport innerhalb des Analyseelements von der Probenauftragszone in Richtung auf die Nachweiszone ermöglicht,
**dadurch gekennzeichnet,**
**daß** es mit einem Denaturierungsreagenz für Nukleinsäuren getränkt ist und die nachzuweisenden Nukleinsäuren auf dem Analyseelement mit Hilfe dieses Denaturierungsreagenzes denaturiert werden.

17. Analyseelement nach Anspruch 16,
**dadurch gekennzeichnet,**
**daß** das Denaturierungsreagenz in trockener Form vorliegt.

18. Analyseelement nach Anspruch 16 oder 17,
**dadurch gekennzeichnet,**
**daß** es ein saugfähiges Materiel enthält, das einen chromatographischen Transport ermöglicht.

19. Analyseelement nach einem der Ansprüche 16 bis 18,
**dadurch gekennzeichnet,**
**daß** es als Teststreifen ausgebildet ist.

20. Analyseelement nach einem der Ansprüche 16 bis 19,
**dadurch gekennzeichnet,**
**daß** es in der Nachweiszone eine zu den nachzuweisenden Nukleinsäuren komplementäre Nachweissonde in immobilisierter Form enthält.

21. Kit zum Nachweis von Nukleinsäuren umfassend
(a) ein Analyseelement nach einem der Ansprüche 16 bis 20 und
(b) weitere zum Nachweis von Nukleinsäuren erforderlichen Reagenzien.

22. Verwendung des Analyseelements nach einem der Ansprüche 16 bis 20 oder des Kits nach Anspruch 21 zum Nachweis von Nukleinsäure-Amplifikationsprodukten.

23. Verwendung nach Anspruch 22 zum Nachweis des Vorhandenseins oder der Menge von ausgewählten Organismen oder Teilen von Organismen in einer Probe.

## Claims

1. Method for the detection of nucleic acids on an analytical element which contains a sample application zone and a detection zone, the analytical element enabling liquid transport from the sample application zone to the detection zone, comprising the steps:
- applying a sample containing the nucleic acids to be detected to the sample application zone,
- detecting the nucleic acids in the detection zone by hybridization with a detection probe,
wherein
the nucleic acids to be detected are not denatured on the analytical element until after the application.

2. Method as claimed in claim 1,
wherein
the nucleic acids are denatured by contact with a denaturing reagent containing a base or/and a chaotropic substance.

3. Method as claimed in claim 2,
wherein
the base is selected from alkali hydroxides and in particular NaOH.

4. Method as claimed in claim 2,
wherein
the chaotropic substance is selected from iodides, acetates, perchlorates, thiocyanates, trifluoroacetates, trichloroacetates or/and guanidinium compounds.

5. Method as claimed in claim 4,
wherein
the chaotropic substance is guanidinium thiocyanate.

6. Method as claimed in one of the previous claims,
wherein
the analytical element contains an absorbent material which enables a chromatographic transport.

7. Method as claimed in one of the claims 1 to 6,
wherein
the analytical element is impregnated with the denaturing reagent before applying the sample.

8. Method as claimed in claim 7,
wherein
the denaturing reagent is present in a dry form on the analytical element.

9. Method as claimed in claim 7 or 8,
wherein
the sample application zone is pre-impregnated with the denaturing reagent.

10. Method as claimed in one of the claims 1 to 6,
wherein
the analytical element is impregnated with the denaturing reagent after applying the sample.

11. Method as claimed in one of the previous claims,
wherein
the analytical element contains a neutralizing reagent between the sample application zone and the detection zone.

12. Method as claimed in one of the previous claims,
wherein
the nucleic acids to be detected are amplification products.

13. Method as claimed in one of the previous claims,
wherein
the detection probe is present in an immobilized form in the detection zone.

14. Method as claimed in one of the previous claims,
wherein
the nucleic acids are detected by means of marker groups.

15. Method as claimed in claim 14,
wherein
indirect marker groups are used.

16. Analytical element for the detection of nucleic acids which contains a sample application zone and a detection zone, the analytical element enabling liquid transport within the analytical element from the sample application zone towards the detection zone,
wherein
it is impregnated with a denaturing reagent for nucleic acids and the nucleic acids to be detected are denatured on the analytical element with the aid of this denaturing reagent.

17. Analytical element as claimed in claim 16,
wherein
the denaturing reagent is present in a dry form.

18. Analytical element as claimed in claims 16 or 17,
wherein
it contains an absorbent material which enables a chromatographic transport.

19. Analytical element as claimed in one of the claims 16 to 18,
wherein
it is designed as a test strip.

20. Analytical element as claimed in one of the claims 17 to 19,
wherein
in the detection zone it contains a detection probe in an immobilized form that is complementary to the nucleic acids to be detected.

21. Kit for the detection of nucleic acids comprising
(a) an analytical element as claimed in one of the claims 16 to 20 and
(b) additional reagents required to detect nucleic acids.

22. Use of the analytical element as claimed in one of the claims 16 to 20 or of the kit as claimed in claim 21 for detecting nucleic acid amplification products.

23. Use as claimed in claim 22 for detecting the presence or the amount of selected organisms or parts of organisms in a sample.

## Revendications

1. Procédé de détection d'acides nucléiques sur un élément d'analyse qui contient une zone d'application de l'échantillon et une zone de détection, l'élément d'analyse permettant un transport de liquide de la zone d'application de l'échantillon à la zone de détection, comprenant les étapes:
- dépôt d'un échantillon contenant les acides nucléiques à détecter sur la zone d'application de l'échantillon,
- détection des acides nucléiques dans la zone de détection par hybridation avec une sonde de détection,
**caractérisé en ce que** les acides nucléiques à détecter ne sont dénaturés qu'après le dépôt sur l'élément d'analyse.

2. Procédé selon la revendication 1, **caractérisé en ce que** les acides nucléiques sont dénaturés par contact avec un réactif de dénaturation contenant une base et/ou une substance chaotrope.

3. Procédé selon la revendication 2, **caractérisé en ce que** la base est choisie parmi des hydroxydes de métaux alcalins, en particulier NaOH.

4. Procédé selon la revendication 2, **caractérisé en ce que** la substance chaotrope est choisie parmi des iodures, des acétates, des perchlorates, des thiocyanates, des trifluoroacétates, des trichloroacétates et/ou des composés de guanidinium.

5. Procédé selon la revendication 4, **caractérisé en ce que** la substance chaotrope est le thiocyanate de guanidinium.

6. Procédé selon l'une des revendications précédentes, **caractérisé en ce que** l'élément d'analyse contient un matériau absorbant qui permet un transport chromatographique.

7. Procédé selon l'une des revendications 1 à 6, **caractérisé en ce que** l'élément d'analyse est imprégné du réactif de dénaturation avant l'application de l'échantillon.

8. Procédé selon la revendication 7, **caractérisé en ce que** le réactif de dénaturation se trouve sous forme sèche sur l'élément d'analyse.

9. Procédé selon la revendication 7 ou 8, **caractérisé en ce que** la zone d'application de l'échantillon est préalablement imprégnée du réactif de dénaturation.

10. Procédé selon l'une des revendications 1 à 6, **caractérisé en ce que** l'élément d'analyse est imprégné du réactif de dénaturation après le dépôt de l'échantillon.

11. Procédé selon l'une des revendications précédentes, **caractérisé en ce que** l'élément d'analyse contient un réactif de neutralisation entre la zone d'application de l'échantillon et la zone de détection.

12. Procédé selon l'une des revendications précédentes, **caractérisé en ce que** les acides nucléiques à détecter sont des produits d'amplification.

13. Procédé selon l'une des revendications précédentes, **caractérisé en ce que** la sonde de détection se trouve sous forme immobilisée dans la zone de détection.

14. Procédé selon l'une des revendications précédentes, **caractérisé en ce que** la détection des acides nucléiques s'effectue par l'intermédiaire de groupes marqueurs.

15. Procédé selon la revendication 14, **caractérisé en ce que** l'on utilise des groupes marqueurs indirects.

16. Élément d'analyse pour la détection d'acides nucléiques, qui contient une zone d'application de l'échantillon et une zone de détection, l'élément d'analyse permettant un transport de liquide à l'intérieur de l'élément d'analyse de la zone d'application de l'échantillon en direction de la zone de détection, **caractérisé en ce qu'**il est imprégné d'un réactif de dénaturation pour acides nucléiques et **en ce que** les acides nucléiques à détecter sont dénaturés sur l'élément d'analyse à l'aide de ce réactif de dénaturation.

17. Élément d'analyse selon la revendication 16, **caractérisé en ce que** le réactif de dénaturation se trouve sous forme sèche.

18. Élément d'analyse selon la revendication 16 ou 17, **caractérisé en ce qu'**il contient un matériau absorbant permettant un transport chromatographique.

19. Élément d'analyse selon l'une des revendications 16 à 18, **caractérisé en ce qu'**il est formé d'une bande de test.

20. Élément d'analyse selon l'une des revendications 16 à 19, **caractérisé en ce qu'**il contient, dans la zone de détection, sous forme immobilisée, une sonde de détection complémentaire des acides nucléiques à détecter.

21. Kit de détection d'acides nucléiques, comprenant:
(a) un élément d'analyse selon l'une des revendications 16 à 20 et
(b) d'autres réactifs nécessaires pour la détection d'acides nucléiques.

22. Utilisation de l'élément d'analyse selon l'une des revendications 16 à 20 ou du kit selon la revendication 21 pour la détection de produits d'amplification d'acides nucléiques.

23. Utilisation selon la revendication 22 pour la détection de la présence ou de la quantité d'organismes ou d'éléments d'organismes choisis dans un échantillon.
